# EUROPEAN PATENT SPECIFICATION

(11) **EP 3 493 730 B1**
(45) Date of publication and mention of the grant of the patent: **16.03.2022**
(21) Application number: 17758259.0
(22) Date of filing: 18.08.2017
(51) Int. Cl.: A61B 5/1455, A61B 5/00, A61B 5/291

(54) **MEASURING APPARATUS AND DEVICE FOR MEASURING CHANGES IN CHROMOPHORE CONCENTRATION**
MESSVORRICHTUNG ZUR MESSUNG VON ÄNDERUNGEN IN DER CHROMOPHORKONZENTRATION
APPAREIL ET DISPOSITIF DE MESURE POUR MESURER LES CHANGEMENTS DE CONCENTRATION DE CHROMOPHORE

(30) Priority: 19.08.2016 GB 201614188
(43) Date of publication of application: 12.06.2019
(73) Proprietor: Gowerlabs Limited, Penge, London SE20 7BW (GB)
(72) Inventor: EVERDELL, Nicholas, Penge London SE20 7BW (GB)
(74) Representative: Flint, Adam
(86) International application number: PCT/GB2017/052451
(87) International publication number: WO 2018/033751

(56) References cited:
- EP-A2- 2 294 973
- US-A- 6 163 715
- US-A1- 2011 208 675
- US-A1- 2012 029 304
- US-A1- 2014 275 888
- US-A1- 2014 275 891
- US-A1- 2015 150 505
- US-A1- 2015 327 777

## Description

### Technical Field

The present disclosure relates to apparatus and devices for measuring changes in concentration of a chromophore in a biological medium.

### Background

There are many applications where it is useful to be able to measure changes in concentration of a chromophore in a biological medium. Known apparatus tends to be bulky and uncomfortable for the subject. Known apparatus is also subject to low levels of signal-to-noise, particularly because the signal strength is often very low whilst noise levels can be very high, which can mask the signals that are to be obtained. It is also difficult or laborious to image the concentration of a chromophore in a biological medium using known devices and apparatus.

US2014275891A1 discloses an optical sensor for providing information about a subject. The optical sensor is placed in proximity to the subject and includes optical sources and optical detectors. The optical sources irradiate the subject with optical signals and the optical detectors detect signals from the subject. Analysis of the detected signals can yield information about the subject.

US2015327777A1 discloses a tissue-monitoring device for selecting, administering or adjusting a treatment based on information obtained by monitoring the tissue. The device includes a substrate for applying to the tissue; a light source supported by the substrate, the light source emitting light of a first frequency for directing the emitted light onto a surface of the tissue; a photodetector carried by the substrate spaced from the light source for detecting scattered light; and a controller.

US2012029304A1 discloses a patient-monitoring system which includes a platform and one or more monitoring modules. A platform and one or more monitoring modules may be coupled to form a communications bus, allowing communication between any of the plurality of coupled devices.

### Summary

According to an aspect disclosed herein, there is provided a measuring apparatus for fitting to an animal body for measuring changes in concentration of a chromophore in the animal body, the measuring apparatus being defined in appended independent claim 1.

The apparatus is convenient for both technicians using the apparatus and the subject to which the apparatus is fitted in use. Examples of the apparatus enable rapid gathering of data from the animal body and facilitate imaging of the region being studied. Having a device controller provided on the devices facilitates modularity of the apparatus as in some examples the devices can more easily be connected to and removed from different locations of the apparatus, and therefore repositioned, as may be required or useful.

The light emerging from said animal body may be for example reflected light or transmitted light, etc., in each case ultimately deriving from the light emitted by the light source at or into the animal body. In general, the "light" may be visible or non-visible light.

In an example, the light sources of the devices are arranged to emit near-infrared light. This may be in the range of for example 600 nm to 1000 nm.

In an example, each of the devices comprises at least two light sources wherein a first light source is arranged to emit light of a first wavelength and a second light source is arranged to emit light of a second wavelength which is different from the first wavelength. In some examples, the use of multiple wavelengths enables signals from different chromophores to be more easily distinguished. By way of a specific example in a specific application, the use of multiple wavelengths enables signals due to the less dominant chromophores to be more easily distinguished from those due to haemoglobin (which is the most dominant chromophore in mammalian tissue), and thus for example provide more complete and accurate information about tissue oxygenation, haemodynamics and metabolism.

In an example, the distance between the at least two light sources is no more than 15% of the distance between each of the at least two light sources and the light detector. The light sources may be provided as components of a light source unit. It is desirable that the light sources are co-located as closely as possible, particularly in the case that the light sources emit different wavelengths, as it can then be assumed or at least approximated with a high degree of accuracy that the measurements sample the same volume of tissue.

In an example, the shared bus has plural connector ports to which the device controllers of at least some of the devices are respectively connected. The shared bus may be for example a shared serial communication bus. According to the invention, at least one of the devices has a connector for removably connecting the device to the shared bus.

In an example, at least one of the devices is connected to the shared bus by flexible conductors.

In an example, the connection arrangement is provided at least in part by a wiring scheme in which the plurality of devices are connected in sequence.

In an example, the connection arrangement is provided at least in part by a wireless connection between at least some of the devices.

In an example, the electrical connector of said at least some of the docks comprises a pogo pin which connects to a corresponding pogo target of a device received in the dock

In an example, the devices comprise a Faraday cage which encloses at least the light detector of the device. Where amplifiers are provided for the light detectors, the Faraday cage of the devices may also enclose the amplifiers.

In an example, each of the devices comprises:
a charge-to-digital converter for the light detector, the charge-to-digital converter having a plurality of amplifiers and an analogue-to-digital converter, wherein:
the plurality of amplifiers are arranged to produce a voltage output; and,
the analogue-to-digital converter is arranged to read the voltage output by one of the amplifiers while another of the amplifiers is accumulating or converting charge from the light detector. This enables continuous operation of the devices. The amplifiers may be for example charge integration amplifiers.

In an example, each of the devices comprises:
a current-to-digital converter for the light detector, the current-to-digital converter having a transimpedance amplifier and an analogue-to-digital converter, wherein:
the transimpedance amplifier is arranged to produce a voltage output; and,
the analogue-to-digital converter is arranged to read the voltage output by the amplifier.

In an example, each of the devices comprises a substrate in which the light source, the light detector and device controller are arranged. The substrate may be transparent to light emitted by the light source.

In an example, each of the devices comprises a plurality of light guides, wherein:
a first light guide is arranged to guide light from the light source to a said animal body; and,
a second light guide is arranged to guide light from a said animal body to the light detector.

In an example, the first light guide is generally cylindrical and arranged around the light source.

In an example, the second light guide is generally cylindrical and arranged around the light detector.

The first and second light guides may be provided as part of a separate component which can be removably placed over the light source and light detector. This enables the light guides to be easily removed for cleaning or replacement (for example, before use on a different subject or biological medium).

In an example, the device controller of each device is a microcontroller, and each device comprises a local memory for storing data received from at least one of a main controller and the light detector. The local memory may be part of the microcontroller and/or provided separately of the microcontroller.

In an example, the device controller is arranged to send signals to the light source in accordance with signals received from a main controller and is arranged to send signals to a main controller in accordance with signals received from the light detector.

In an example, the signals include at least one of light source intensity instructions, clock signals and data signals.

In an example, at least one device comprises an electrode arranged to measure electrical signals produced in a medium to non-invasively monitor electrical activity in the medium. The device may be used to obtain for example simultaneous electroencephalography (EEG) measurements.

In an example, the apparatus comprises:
a main controller for sending signals to and receiving signals from the devices; and,
the shared bus is arranged to relay signals between the main controller and the devices, the signals including at least one of light source intensity instructions, clock signals and data signals;
the main controller being arranged to generate a system clock and initiate communication with the devices.

There is also described herein a device for use with measuring apparatus as described above for measuring changes in concentration of a chromophore in a biological medium, the device comprising:
a light source for emitting light towards a biological medium;
a light detector for detecting light emerging from said biological medium; and,
a device controller for receiving signals from and sending signals to a main controller.

Such a device is in effect a self-contained unit, at least to a large extent. Having a device controller provided on the device facilitates modularity of the apparatus with which plural such devices may be used in practice.

In an example, the light source is arranged to emit near-infrared light. This may be in the range of for example 600 nm to 1000 nm.

In an example, the device comprises at least two light sources wherein a first light source is arranged to emit light of a first wavelength and a second light source is arranged to emit light of a second wavelength which is different from the first wavelength.

In an example, the device has a connector for removably connecting the device to a bus. The bus may be for example a serial communication bus.

In an example, the device has a wireless transceiver for wirelessly connecting the device to another device as described above. In an example of this, the device may have its own power source, such as a battery, which may be a rechargeable battery or a non-rechargeable battery, in effect enabling the device to be a self-contained measuring device.

### Brief Description of the Drawings

To assist understanding of the present disclosure and to show how embodiments may be put into effect, reference is made by way of example to the accompanying drawings in which:
Figure 1 shows a schematic view of an example of a system for measuring changes in concentration of a chromophore in a biological medium;
Figure 2 shows a schematic perspective view of an example of a device for use with apparatus for measuring changes in concentration of a chromophore in a biological medium;
Figure 3 shows a schematic view of a face of the device of Figure 2;
Figure 4 shows a schematic lateral cross-sectional view of an example of a light guide for use with apparatus for measuring changes in concentration of a chromophore in a biological medium;
Figure 5 shows another schematic view of an example of apparatus for measuring changes in concentration of a chromophore in a biological medium;
Figure 6 shows a side view of another example of a device for use with apparatus for measuring changes in concentration of a chromophore in a biological medium assembled with a dock and a light guide arrangement;
Figure 7 shows a cross-sectional view on VII-VII of the assembly of Figure 6;
Figures 8A to 8C show a perspective view of the device, dock and light guide arrangement of Figure 6 in disassembled form;
Figure 9 shows an exploded view of the device of Figure 8A;
Figures 10A and 10B show respectively perspective views from above and below of an assembled circuit board of the device of Figure 8A;
Figure 11 shows an exploded view of the dock of Figure 8B; and
Figure 12 shows an example of a cap or headgear having docks of Figure 8B for receiving devices of Figure 8A and light guides of Figure 8B.

### Detailed Description

As mentioned above, there are many applications where it is useful to be able to measure changes in concentration of a chromophore in a biological medium. For example, it can be useful to measure changes in concentration of a chromophore in an animal body, including in particular a human body, where chromophores may be found, for example, in blood flowing through the brain, or heart, or skeletal muscle, etc., etc.

As one particular example, apparatus for performing functional neuroimaging which aim to measure regional brain activity are known. Functional near-infrared spectroscopy (fNIRS) is a known non-invasive technique for providing information on the vascular and metabolic response to brain activity. The chromophores may be or include for example oxy-haemoglobin, deoxy-haemoglobin and oxidised cytochrome c oxidase. Oxy-haemoglobin and deoxy-haemoglobin are found in the blood. Oxidised cytochrome c oxidase can be found in tissue. Measurement of the changes in concentration of these chromophores may offer insight into, for example, regions of activity in the brain.

Known fNIRS systems typically involve coupling large arrays of optical fibres or fibre bundles to the scalp. Imaging over an entire scalp typically requires around 30 discrete source positions and a similar number of detector positions, which often results in an array that is bulky and heavy, and that severely restricts movement. More recently, portable systems have been developed which employ sources and detectors in direct contact with the scalp, with the analogue signal being transferred via long electrical cables to a controller module. One problem with this approach is that long analogue cables can introduce noise into the measurement. It is also difficult or laborious to image the concentration of the chromophores with known fNIRS systems. Patients or other people or animals who are subject to such studies are, inevitably, often reluctant to be subjected to extended periods of study, or are even incapable of being studied for an extended period.

Examples of apparatus 100 described herein may be used to measure changes in concentration of a chromophore in a biological medium. In turn, this may enable changes in concentration of a chromophore in a biological medium to be monitored, over time. Furthermore, examples of apparatus 100 described herein may be used to enable images of concentration of a chromophore in a biological medium to be produced, and to be produced in real time, which is of significant value to practitioners.

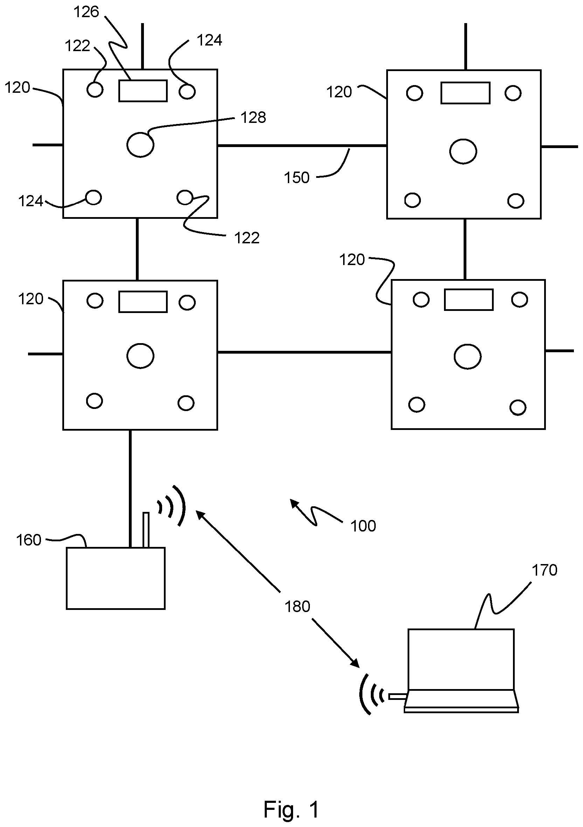

Referring to Figure 1, there is shown a schematic view of an example of an apparatus 100 for measuring changes in chromophore concentration in the body. The apparatus 100 in this example has at least one device 120 and a bus 150, which in use is in communication with a base unit 160 which in turn is in communication with a data recorder such as a computer 170. The base unit 160 may act as a "hub" or master or main controller for the apparatus 100.

In typical implementations, the apparatus 100 has plural devices 120, which are identical or at least have substantially the same functionality, and the bus 150 is a shared bus 150 to which all of the devices 120 are connected. In the figure, only a portion of the apparatus 100 and four devices 120 are shown. In practice, there will typically be say around 20 to 30 or more such devices 120. The devices 120 are connected to each other by the shared bus 150, which also connects to the base unit 160. In the example shown, the base unit 160 is connected to the computer 170 by a wireless link 180. The base unit 160 may have its own power source, such as a battery, which may be a rechargeable battery or a non-rechargeable battery. The power source of the base unit 160 may power the components of the base unit 160 and optionally also the devices 120 (though the devices 120 in some examples may have their own power source, such as a rechargeable battery or a non-rechargeable battery). A wireless link 180 between the base unit 160 and the computer 170, and providing the base unit 160 and devices 120 with their own power source(s), is more convenient as it means that the combination of the devices 120 and the base unit 160 (which itself may be relatively small and lightweight) can be effectively untethered and therefore made both wearable and portable. This is convenient for both technicians using the system and the person or other subject under study, who is able to move around relatively freely if desired. Nevertheless, a wired connection to the computer 170 and/or providing some external power supply for powering the base unit 160 and/or the devices 120 may be used instead in other implementations. The devices 120 may be in the form of blocks or "tiles", which facilitates the modularity of the apparatus 100 as individual devices 120 can be added or removed as desired or necessary.

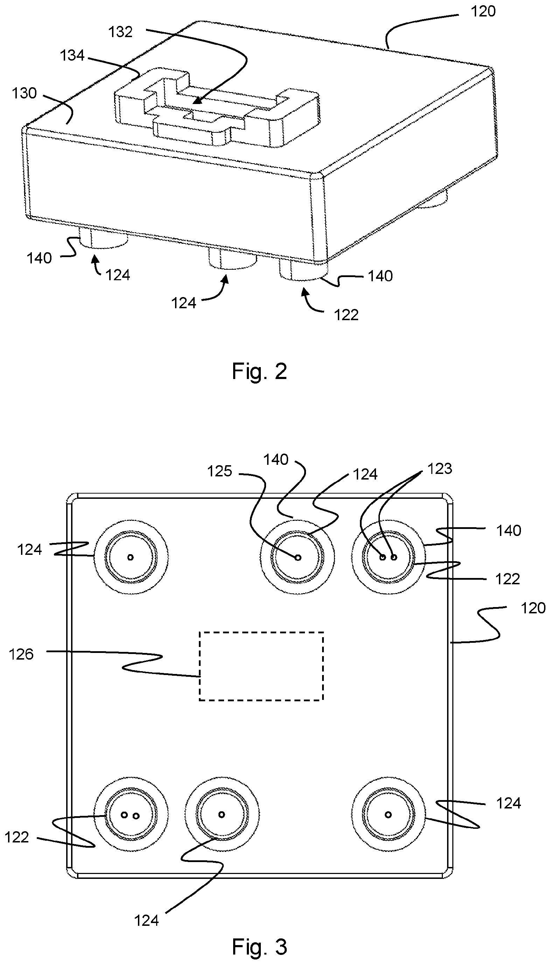

Referring additionally to Figures 2 and 3, there are shown schematic views of an example of a device 120. In this example, the device 120 is generally cuboid, having a rectangular or square cross-sectional shape. The device 120 has at least one light source unit 122 and at least one light detector unit 124. In the specific example shown in these figures the device 120 has two light source units 122 and four light detector units 124. Each light source unit 122 has or contains at least one light source 123 for emitting light. In an example a light source unit 122 has a plurality of light sources 123. In a specific example, each light source unit 122 has two light sources 123. Each light source 123 may emit light having a wavelength in the range of for example around 600 nm to 1000 nm. The light sources 123 may emit light having different wavelengths. In an example, each light source unit 122 has a first light source 123 which can emit light at a first wavelength and a second light source 123 which can emit light at a second wavelength. The light sources 123 may be for example light-emitting diodes (LEDs). The light detector units 124 are arranged to detect light having a wavelength that is emitted by the light sources 123. In the example shown, the light source units 122 and light detector units 124 are mounted in or supported by a substrate 130 which provides the bulk of the body of the device 120. The substrate 130 may be for example black pigmented silicone.

In use, the light sources 123 emit light towards a medium in which a chromophore is present. In a specific example, the medium may be a human head, but may also be other body parts of a human or other animal. As mentioned above, the light sources 123 may emit at a number of different discrete wavelengths. In the specific example of fNIRS, it has been found to be useful to use light of wavelengths around 780 nm and 850 nm to monitor changes in oxyhemoglobin and deoxyhemoglobin concentrations respectively, though different wavelengths may be used. More generally and by way of further example, the shorter wavelength may be in the range of around 650 nm to 780 nm and the longer wavelength may be in the range of around 820 nm to 900 nm.

To accurately interpret data with the apparatus 100, it is desirable to assume that the measurements sample the same volume of tissue. It is therefore preferred that the sources of the discrete wavelengths are co-located as closely as possible. The separation between the light sources 123 in the light source units 122 is therefore preferably small or even negligible compared to the distance between the light source units 122 and the light detector units 124. In an example, the distance between the light sources 123 of a light source unit 122 is not more than 15% of the distance between the light source unit 122 and the light detector unit(s) 124. In one example, the distance between the light sources 123 of a light source unit 122 is not more than 10% of the distance between the light source unit 122 and the light detector unit(s) 124. In another example, the distance between the light sources 123 of a light source unit 122 is not more than 5% of the distance between the light source unit 122 and the light detector unit(s) 124. In an example, the distance between the light sources 123 in a light source unit 122 may be less than a few millimetres whereas the distance between the light source unit 122 and the light detector unit(s) 124 may be up to a few centimetres. In a specific example, the distance between the light sources 123 in a light source unit 122 of a device 120 may be between around 0 mm to 1 mm, such as around 0.3 mm whereas the distance between the light source unit 122 and the light detector unit 124 of a device 120 may be around 8.5 mm, or at least may be a minimum of around 8.5 mm. (Where there are plural devices 120, the minimum distance between a light source unit 122 of a device 120 and a light detector unit 124 of an adjacent device 120 may be for example around 10 mm to 25 mm.)

For some applications, the arrangement of the light source units 122 and light detector units 124 may be chosen so that the (shortest) distance between a light source unit 122 and a light detector unit 124 is small, such as a few millimetres, such as around 3 mm or less. A light source unit 122 and a light detector unit 124 may be closely located and may in some cases be practically co-located.

The light sources 123 in the light source units 122 may be driven by for example a multiplexed voltage-controlled current source (VCCS). This enables accurate control of the current flowing through the light source 123, which is closely related to its optical intensity. An analogue multiplexer is used instead of discrete transistors to switch between light sources 123. The multiplexing VCCS scheme is more compact and flexible than traditional resistor/op-amp combinations.

Each light detector unit 124 has at least one light detector 125, which may be for example a photodiode, for detecting light. In the example shown, each light detector unit 124 has a single light detector 125. In a specific example, the four photodiode detectors 125 of the light detector units 124 of the device 120 are coupled to respective channels of a four-channel charge-to-digital converter which includes two charge integrators per channel and at least one analogue-to-digital converter (ADC) and which may be provided on the device 120. In a specific example, the device 120 has two ADCs. The charge integrators (also known as charge amplifiers or charge integration amplifiers) integrate the photodiode current generated by the incident light to output a voltage. The use of two parallel charge integrators per channel enables one charge integrator to integrate the generated photodiode current while the output of the other charge integrator is being digitized by an ADC. This facilitates continuous operation of the device 120 without any dead time. Moreover, because the analogue signals are digitized early in the signal path (that is, effectively as a first step as the analogue signals are output by the light detector units 124), the signal-to-noise ratio is improved.

In a specific example, the charge integrators are provided as part of an analog-to-digital converter, which may be for example the DDC114 of the Texas Instruments DDC family. In another example, the charge integrators are part of the DDC118 of the Texas Instruments DDC family. The light detectors 125 are directly connected to the high impedance analogue inputs of the DDC114/DDC118. Providing the pairs of charge integrators and their associated ADC within one integrated circuit leads to a superior signal-to-noise ratio as compared with the use of discrete elements.

As an alternative to or in addition to the use of charge integrators, current-to-voltage converters can be used for photodiode signal amplification, in the form of transimpedance amplifiers. In a specific example, a Texas Instruments OPA380 may be used. In a specific example a transimpedance amplifier may then be connected to its own ADC. In another example, more than one transimpedance amplifier may be multiplexed to a single ADC that has multiple input channels.

The device 120 has a device controller 126. The device controller 126 in an example is a microcontroller. The device controller 126 is arranged to send signals originating in the light detector units 124 to the base unit 160 via the bus 150. The device controller 126 is arranged to receive signals from the base unit 160 via the bus 150. As mentioned, the base unit 160 is in communication with the computer 170. In this way the computer 170 may communicate with the device 120. The base unit 160 may send signals to the device 120 which include at least one of light source intensity instructions, clock signals and data signals. In the case that the light sources 123 are LEDs, the light source intensity instructions may be for example LED status commands which control switching the LEDs on and off as well as controlling the intensity of their illumination. The clock signals enable the base unit 160 to synchronise one or more components of the apparatus 100.

In a specific example the device 120 has a local memory store for the device controller 126. The local memory store may be a part of the device controller 126, particularly in the case that the device controller 126 is a microcontroller. Additionally or alternatively the local memory store may be provided as separate memory. In the specific example wherein the local memory store is provided as a separate memory, the device controller 126 is in communication with the local memory store. Either way, the device controller 126 may store in the local memory information received or obtained from the light detector units 124. The device controller 126 may additionally or alternatively store instructions for the light source units 122 in the memory store. The information and instructions may be stored locally to the device 120.

Referring specifically now to Figures 2 and 3, in this example the device 120 shown has a connector 132 for removably connecting the device 120 to the bus 150. The connector 132 in this example is provided internally of a lip 134 in the substrate 130 of the device 120. The connector 132 can receive a portion of the shared bus 150 to connect the device 120 to the bus 150. In the example shown the connector 132 is on the opposite face of the device 120 to the light source units 122 and light detector units 124. This helps prevent the shared bus 150 from physically interfering with the operation of the light source units 122 and light detector units 124.

The device 120 may have a flexible or a so-called "rigid-flex" printed circuit board. A rigid-flex circuit is a hybrid construction of a circuit on rigid and flexible substrates which are laminated together to form a single structure. In this example, the rigid-flex printed circuit board has flexible conductors which may connect the device 120 to, for example, the base unit 160. In this example, the device 120 therefore does not need a connector 132 of the type described above. The flexible conductors of the rigid-flex printed circuit board may alternatively or additionally be used to connect one device 120 to another device 120. In an example, the entire printed circuit board of the device 120 is enclosed within a Faraday cage, which reduces or eliminates the susceptibility of the device 120 to external electromagnetic noise. A specific example of an arrangement for a Faraday cage will be discussed further below.

In an example the light source unit(s) 122 and light detector unit(s) 124 of the device 120 are each encapsulated in a protective polymer, such as for example optically transparent silicone or UV-curing epoxy resin. In an example the protective polymer is transparent to light emitted by the light sources 123.

In an example, the one or more light sources 123, such as LEDs, within a light source unit 122 may be encapsulated in a protective polymer, such as optically transparent silicone or UV-curing epoxy resin. This may be carried out as follows. A silicone rubber mask may be made to create a cavity for dispensing the liquid epoxy. As silicone rubber is inherently hydrophobic, a chemical bond to the epoxy resin is prevented and the mask is easily removed when the epoxy is fully cured. The mask may be produced using an open cast mould made out of 0.5 mm thick laser-cut acrylonitrile butadiene styrene (ABS). The mould may be lightly sprayed with a releasing agent before platinum cure silicone polymer is poured in. The one or more light sources 123, such as LED dies, for each light source unit 122 may then be positioned in the silicone, which is then cured. Alternatively, a Perspex (TM) mask may be used to form the silicone mask. In a specific example, the Perspex mask is around 1.5 mm thick. The silicone mask is cured and then pushed against the LEDs. The window within the silicone mask may then be filled with UV-curing epoxy.

The LEDs may be for example near-infrared AlGaAs pn-junction semiconductors. The LED dies have the n-side (cathode) of the LED facing upwards, while their opposite p-side (anode) is metallized for adhesion to the substrate. All the dies are attached to a square gold-coated substrate with dimensions of 2 mm × 2 mm. This area is used as the common anode, and each die can be individually addressed by passing a current through its cathode. Initially, the substrate is coated with a thin layer of a thermally-curable electrically-conductive paste. The paste creates an electrical connection for the rear surface (anode) of the dies and provides mechanical fixation once it is cured.

As shown in Figure 3, light guides 140 are arranged around at least some and preferably each of the light source units 122 and the light detector units 124. The light guides 140 create an optical interface between the light source units 122 and the light detector units 124 and the body in which changes in concentration of a chromophore are being monitored. More specifically, the light guides 140 provide a medium through which light may travel between the device 120 and the body. The light guides 140 may bypass hair on, for example, a scalp or chest so that emitted light does not pass through the hair, which could otherwise reduce the signal-to-noise ratio of the acquired signal. The light guides 140 may be formed of a semi-flexible material which is comfortable when in contact with a subject. In an example the light guides are generally cylindrical. The cross-sectional shape of the light guides may be for example circular or elliptical or polygonal, such as square or other regular or irregular shape.

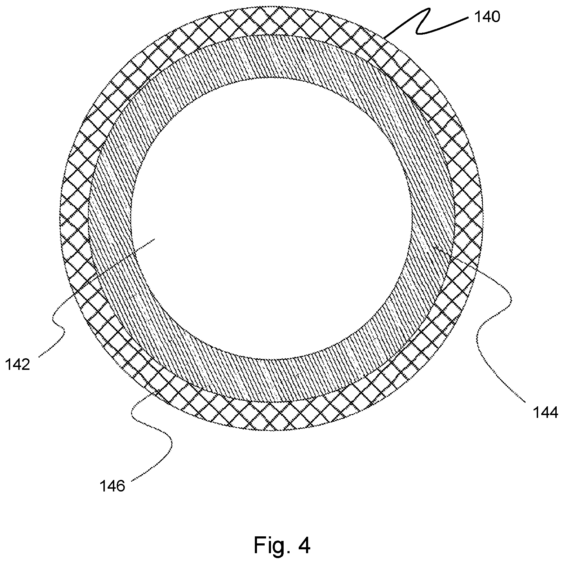

Referring now to Figure 4, there is shown a specific example of a light guide 140. The light guide 140 has an inner core and at least one coating layer. In the example shown the light guide 140 has an inner core 142, an inner coating 144 and an outer coating 146.

In an example the inner core 142 is a light transmission medium and is transparent to light emitted by the light sources 123. The inner core 142 may be made of, for example, optically clear silicone polymer. The inner core 142 may be made by for example filling an acrylic (poly(methyl methacrylate) or PMMA) tube of a predetermined diameter with liquid silicone. The filled tube is placed in an oven, or similar curing environment, for the silicone to cure. Once cured, the acrylic tube is broken and the silicone core is removed. In an example, the light guide 140 may be formed of glass, for example borosilicate glass.

The inner coating 144 is a light reflective medium having a reflective surface to channel rays of light within the light guide 140. The inner coating 144 may be formed of transparent silicone mixed with a white, or substantially white, pigment to create a diffusive reflective surface. The white pigment may be a white powder from a metal oxide such as for example titanium dioxide. The silicone and pigment is highly scattering to the light and diffusively reflects light at its surface. The inner coating 144 ensures efficient transmission of light from the light sources 123 to the medium in which changes in a concentration of a chromophore are being measured and from the medium to the light detector unit 124.

The outer coating 146 is an opaque medium to prevent light from outside the light guide 140 passing into the light guide 140. The outer coating 146 also prevents light transmission from within the light guide 140 to the outside of the light guide 140. The outer coating 146 may be formed of transparent silicone mixed with a black, or substantially dark, pigment to provide an opaque surface. The black pigment may be for example charcoal powder.

The light guides 140 do not need to be polished during production as the light guides 140 can be cut with a sharp tool, such as a razor blade. The light guides 140 may be made of a material such that the light guides 140 do not get easily scratched, snapped or chipped in use, which is important given the intended application of the light guides 140. The silicone in the inner core 142, the inner coating 144 and the outer coating 146 of the light guide 140 may be formed of the same silicone in order to maximize adhesion strength. This facilitates strong bonding between the three parts of the light guide 140.

In the case that the light guide 140 is made of a polishable substance, for example glass or PMMA (poly(methyl methacrylate)) or the like, one end of the light guide 140 may be polished to achieve a flat surface for improving optical transmission. This will increase the transmission of emitted light into the medium in which changes in a concentration of a chromophore are being monitored. The other end of the light guide 140 may be coupled to a light source unit 122 or a light detector unit 124 using an optical grade UV cured adhesive.

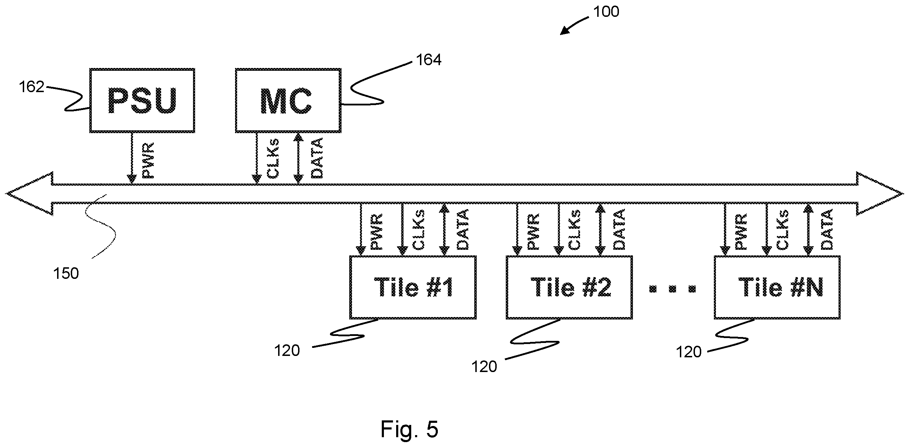

Referring now to Figure 5, there is shown a schematic drawing of the apparatus 100 to illustrate the connections to the bus 150. In the example shown, the bus 150 is a shared bus 150. In yet another example the bus is a serial communication bus 150. The bus 150 variously carries one or more of power, light source intensity instructions, clock signals and data signals to and from the base unit 160, which in this example includes a power supply unit 162 (which may be for example a battery, which may be rechargeable or non-rechargeable) and a main controller 164. In a specific example the bus 150 is an inter-integrated circuit I²C shared serial bus. A standard I²C bus may be used utilising two-way addressable communication. Buses using different protocols for addressing the individual devices 120 may be used instead.

In an example the bus 150 is a single "highway" connecting each device 120 to each other device 120 in a linear manner, starting at the base unit 160, passing through each device 120 and terminating at the device 120 furthest from the base unit 160. In another example the bus 150 is in the form of a "two-dimensional" mesh. A mesh configuration may have a more even power distribution among the devices 120. Both the highway and mesh configurations have no upper limit as to the number of devices 120 which can be connected to the bus 150. That a large number of devices 120 can be connected, and the information from each device 120 can be easily processed, enables a large area to be covered and monitored. This, in turn, enables easier investigation of, for example, body parts of animals with large surface areas and enables more rapid gathering of data, which in turn facilitates imaging of the chromophores in the biological medium.

In another example, the bus 150 is not a physical, wired bus 150 arranged between the devices 120 but is instead a wireless connection between the devices 120. Such a wireless connection may be for example via a Bluetooth ^{™} connection or another suitable wireless network connection. In an example of this, each of the devices 120 may have its own power source, such as a battery, which may be a rechargeable battery or a non-rechargeable battery. This in effect enables each device 120 to be a self-contained measuring device.

In an example, the devices 120 in the apparatus 100 may be connected at least in part by a wiring scheme in which the devices 120 are connected in sequence or in a ring. In a specific example, the devices 120 may be connected by "daisy chaining" the devices 120 to each other. In this example the data lines are connected in series rather than in parallel (such as in I²C). The data is passed from one device 120 to another device 120 and so the packet of data increases or decreases in size depending on whether the device 120 is transmitting or receiving. An advantage of this arrangement is that the data rates are high in comparison with other wiring schemes. This is particularly advantageous if there is a large number of devices 120 that are connected. Specific examples of implementations for daisy chaining include the daisy-chained JTAG (Joint Test Action Group) IEEE (Institute of Electrical and Electronics Engineers) Standard 1149.1) and the WS28XX series of RGB LEDs by Shenzhen Worldsemi Technology Co., Ltd.

A description of an example of the operation of the system now follows. A square electrical waveform is generated by the base unit 160, and the rising and falling edges of the pulses determine the start and end times of the charge integration for each detector channel. This square pulse is the main clock for the system. Each device 120 and the base unit 160 use a common clock. The frequency of the common clock determines the length of integration time for the controller 126 of each device 120. This integration time may be a global parameter for all devices 120. In a specific example, the base unit 160 and the device controller 126 each use or are a Cypress PSoC (programmable system-on-chip) microcontroller. This enables the use of dedicated logic for clock generation and management which is not possible with traditional microcontrollers.

A square waveform is preferred, but in general the waveform may be other than a square wave and does not need to be symmetrical. For example, an asymmetric pulse with a small duty cycle (e.g. 10% or so) may be used. This enables consecutive short and long integration times. Combining the results from both short and long integrations increases the dynamic range of the measurement system, whilst incurring only a small penalty to the overall sample rate of the system.

After the integration time has elapsed, the device controller 126 receives data signals from the analog-to-digital converter (e.g. the DDC114 mentioned above) and stores them in local memory. Then the device 120 waits for the I²C command packet addressed to the specific device 120 from the base unit 160. The command from the base unit 160 includes device operational parameters such as light source intensity instructions for the LEDs to be used during the next integration period. The LED intensity instructions, or control signals, determine if the LEDs on the device 120 should be ON or OFF during the next integration cycle. Once the command is received by the device 120, the base unit 160 sends an I²C command addressed to the same device 120 in order to retrieve data signals stored in the local memory. These packets may include information from the photodiodes of the light detector units 124 available on the device 120. In addition, data from peripheral components (e.g. temperature and motion sensors) may be included in the data signals. Once a particular data signal is received by the base unit 160, the address will increment and the next device 120 will be targeted. This process is repeated until all the devices 120 have been addressed. In an example the bus 150 is an electrically shared bus 150 which uses I²C standard bus hardware. The bus 150 may also be a single cable (i.e. a bundle of wires carrying clock and data signals, as well as electrical power) and can be shared amongst all the devices 120 without any physical preference of the order in which they are attached to the bus 150. This makes the system flexible and modular.

The use of the bus 150 and the modularity of the apparatus 100 as a whole enable the simultaneous acquisition of data within and across all devices 120, regardless of their physical layout. An intra-device measurement involves a measurement of light from a light source 123 by a light detector 125 residing on one device 120. An inter-device measurement involves measurement of the light from a light source 123 on one device 120 by a light detector 125 on another device 120. Although examples of the apparatus 100 will in general be made up of many devices 120, from the overall system perspective, all the light sources 123 and light detectors 125 are accessible simultaneously, regardless of on which device 120 they reside. In principle and in general, every light detector 125 can make measurements of light emitted by every light source 123. Since all photodetection is performed simultaneously, there is no significant scanning rate penalty when the number of light detectors 125 is increased; it is only necessary to increase the data throughput rate on the bus 150 itself. Therefore, the system provides a significant increase in speed and efficiency over known systems for both data collection and retrieval. The (very) large number of light source-detector pairs that can be obtained with this system provides a great improvement in data quality. This is particularly relevant in imaging applications.

Many existing systems only have a sparse arrangement of light sources 123 and light detectors 125, and the light detectors 125 communicate with their nearest-neighbour light sources 123 only, giving a limited range of light source-detector spacings. With examples of the present system, data with a wide range of spacings and with large numbers of overlapping channels can be collected. The modularity of the present system increases this functionality even further, enabling movement between devices 120, to vary the distribution of light source-detector spacings.

These advantages result in better quality and higher resolution images than are achievable with known wearable systems. The images produced using the apparatus 100 can show changes in chromophore concentration in the mediums being monitored.

Furthermore, in some examples, the use of a single cable bus 150 leads to a significant reduction in the total amount of cabling required, in comparison with known systems which typically have separate cables for each light detector. This results in the weight of the system being considerably less than known systems. This, in turn, increases the wearability of the apparatus 100 and the comfort for a user, and reduces cost on cabling.

During use of some examples of the system, the light sources 123, which in an example are LEDs, are illuminated for the period of the integration time. However, with the introduction of an independent clock for the LED light sources 123, their on-time can be significantly reduced, for example to the order of microseconds. This enables accumulation of less charge on the analog-to-digital integrators, which avoids saturation in situations where attenuation of the transmitted light is low.

Moreover, this implementation may use a combination of a charge integrator and a digital timer-counter simultaneously to measure the light intensity. If the attenuation is high, the charge integrator will not saturate and a readout as described above will be obtained. If the attenuation is low (e.g. because of small source-detector separations) the charge integrator may saturate. However, in that case, the timer-counter may be arranged to stop once the saturation is observed. The time-to-saturation is then proportional to the intensity of the light received. In this way the dynamic range of the measurement system can be greatly increased.

The devices 120 in some examples may all have the same hardware and the same software and/or firmware other than the address code for the devices 120. Each device 120 has a unique address so it can be individually addressed by the base unit 160, which is made possible by virtue of the devices 120 each having their own controller 126. Devices 120 can therefore easily be added to or removed from the bus 150, and this can be done even while the apparatus 100 is in use. If the base unit 160 addresses a device 120 which is not connected to the bus 150, the command will fail and no signals will be returned. The base unit 160 will then address the next device 120. The physical order in which devices 120 are connected to the bus 150 is therefore irrelevant, which is convenient for technicians using the system in practice. In an example, each device 120 has a 2-pin programming feature. (The programming pins are located on the device 120 and are not part of or connected to the shared bus 150.) The 2-pin programming feature on each device 120 facilitates the programming of each device controller 126.

At least some of the devices 120 may also have an electroencephalography (EEG) electrode 128, which can be used for non-invasively measuring electrical potentials on the scalp resulting from underlying neuronal activity. EEG is known as a complimentary technique to fNIRS. The EEG electrode 128 may be controlled by the device controller 126. Providing EEG electrodes 128 on the same device 120 as the light sources and detectors for monitoring changes in concentration of a chromophore enables EEG measurements to be obtained at the same time, which can provide further useful information.

The examples described herein are to be understood as illustrative examples of embodiments of the invention. Further embodiments and examples are envisaged.

For example, instead of using a protocol that enables the devices 120 to be addressed individually, a time division multiple access scheme may be used in which each device 120 is accessed sequentially in turn. This may enable higher data transfer speeds to be achieved, which in turn allows a larger number of devices 120 to be used. As another alternative, a daisy chained bus (described above) using a serial shift register may be used. In this example, the physical medium is not shared but is connected to each device 120 in turn. Hence, there is a data-in connection and a data-out connection at each device 120. Although in this example the devices 120 cannot be removed while the system is running, the serial shift register ensures high data rates, and so can support a larger number of devices 120.

As another example, a combination of a small photodiode detector 125 and a large photodiode detector 125 within the same detector unit 124 can be used to improve the dynamic range of the apparatus 100. The added dynamic range is proportional to the ratio of the detection areas of the large and small photodiode detectors 125.

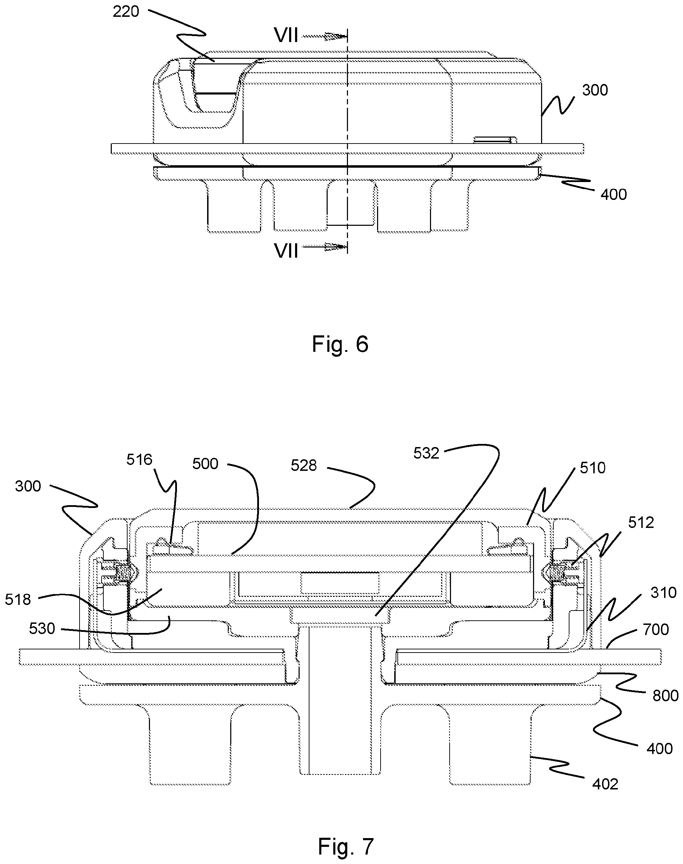

Another example of a device for use with apparatus for measuring changes in concentration of a chromophore in a biological medium, and a dock and a light guide arrangement for use with the device, will be described with reference to Figures 6 to 11. Much of the description of the various features and options of the first example discussed above is applicable to the second example and will therefore not be repeated in detail here. Likewise, many of the various features and options of the second example discussed below are applicable to the first example and can be incorporated therein.

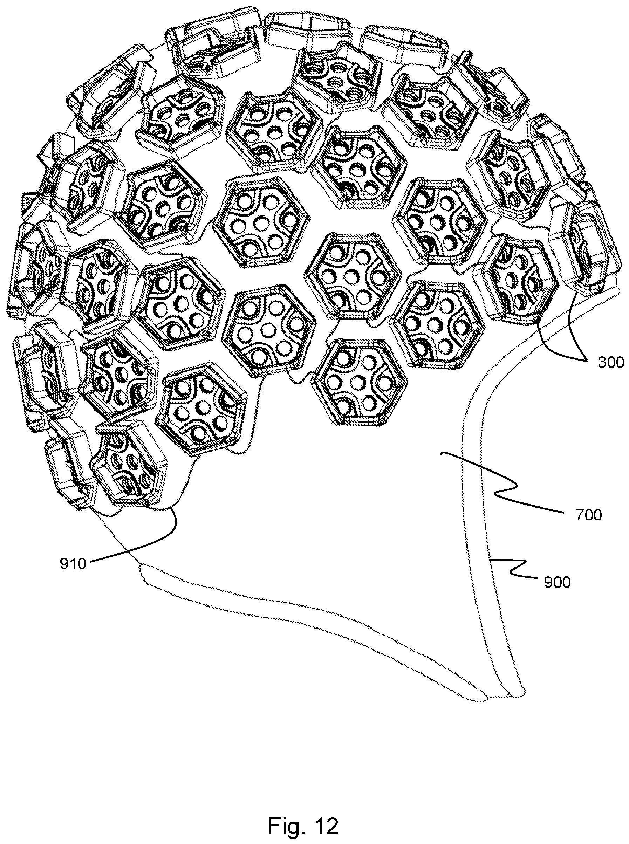

Figure 6 shows a side view of an assembly of the second example of a device 220 for use with apparatus for measuring changes in concentration of a chromophore in a biological medium assembled with a dock 300 and a light guide arrangement 400. Figure 7 shows a cross-sectional view on VII-VII of the assembly of Figure 6. As will be discussed further below, in an example, the device 220 is removably received in and connectable to the dock 300. The dock 300 itself may be one of a number of docks 300, each for receiving a respective device 220 and which may be carried on or fixed to some carrier. A specific example of this, in the form of a cap or headgear, will be discussed with reference to Figure 12.

The device 220 in this example is hexagonal in shape/footprint. Correspondingly, the dock 300 into which the device 220 is fitted in use is also hexagonal in shape. The light guide arrangement 400 is also generally hexagonal in shape. The hexagonal shape is of advantage as it makes it easier to cover a particular area or region that is to be subject to study. In an example, the side of each hexagon of the device 220 is 14 mm. A smaller size is preferred as it enables more of the devices 220 to be used to cover a particular area or region, which enables the resolution of the imaging to be greater.

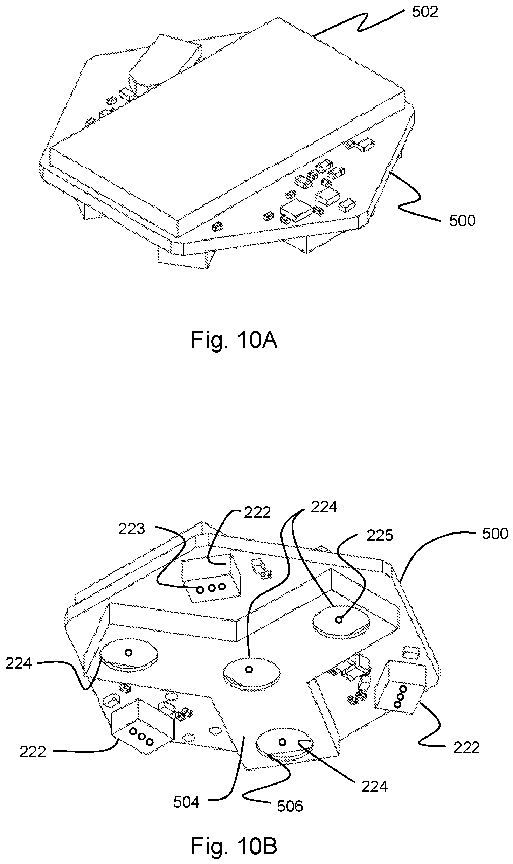

As shown most clearly in Figure 10B, the device 220 has at least one light source unit 222 and at least one light detector unit 224. In the specific example shown, the device 220 has three light source units 222 and four light detector units 224. The three light source units 222 are symmetrically arranged on the device 220, in this example being located towards alternating corners of the hexagon. The four light detector units 224 are likewise symmetrically arranged on the device 220, in this example with three being located towards the other alternating corners of the hexagon and the fourth being located centrally of the hexagon.

Each light source unit 222 has or contains at least one light source 223 for emitting light. In an example a light source unit 222 has a plurality of light sources 223. In the specific example shown, each light source unit 222 has three light sources 223. Each light source 223 may emit light having a wavelength in the range of for example around 600 nm to 1000 nm. The light sources 223 may emit light having different wavelengths. That is, in an example, each light source unit 222 has a first light source 223 which can emit light at a first wavelength, a second light source 223 which can emit light at a second wavelength, and a third light source 223 which can emit light at a third wavelength, each wavelength being different. In an example, the three wavelengths might be 735 nanometres, 810 nanometres and 850 nanometres. The light sources 223 may be for example light-emitting diodes (LEDs). In use in an example, each of the light sources 223 is powered in turn such that, at least within a particular light source unit 222, only one light source 223 is emitting light at a time.

Each light detector unit 224 has at least one light detector 225, which may be for example a photodiode, for detecting light. In the example shown, each light detector unit 224 has a single light detector 225. The light detectors 225 are arranged to detect light having a wavelength that is emitted by the light sources 223. In use in an example, each light detector 225 is continually able to detect light.

In the example shown, the light source units 222 and light detector units 224, and specifically the light sources 223 and light detectors 225 contained therein, are mounted directly on a printed circuit board (PCB) 500. An advantage of mounting the light source units 222 and light detector units 224, and specifically the light sources 223 and light detectors 225 contained therein, directly on the PCB 500 is that it avoids (relatively) long connecting wires from the PCB to the light sources 223 and light detectors 225, which therefore reduces noise and interference. The light detector units 224 are arranged on the PCB close to their respective amplifier circuits, which again helps to keep down noise arising in the device 220. (Examples of arrangements for the amplifier circuits are discussed above in relation to the first example.)

A Faraday cage encloses the light detector units 224 and their amplifier circuits (the amplifier circuits therefore not being visible in the drawings for this example). The Faraday cage consists of two main parts, a first part 502 on the top of the PCB 500 and a second part 504 on the bottom of the PCB 500. In an example, the Faraday cage can be made from copper coated with a thin layer of tin. The close proximity of the detectors units 224 and their respective amplifier circuits together with the Faraday cage 502, 504 provides a low noise detection system, which maximises the signal-to-noise ratio of the device 220. As mentioned above, the signal strength is often very low whilst noise levels can be very high, and so increasing the signal-to-noise ratio of the device 220 is very important.

In an example, the Faraday cage part 504 on the underside of the PCB 500 has holes 506, which may be cut into the part 502 during manufacture, to allow light to reach the detector units 224. The entrances to these holes 506 in one example are covered with a layer of material which is transparent to the light being used and which is electrically conductive, to maintain the integrity of the Faraday cage 502, 504 whilst still allowing light to reach the detector units 224. An example of a suitable material is indium tin oxide.

In an example, the PCB 500 has a number of conducting pads (not shown) which enable connection to a number of "pogo targets" 510, which in turn enable connection to a number of "pogo pins" 512 mounted on a suitable docking system, such as the dock 300 discussed further below. (As is known, a "pogo pin" is a device used in electronics to establish a (usually temporary) connection between two printed circuit boards, devices, etc. A pogo pin is typically in the form of a slender cylinder containing two sharp, spring-loaded pins.) Spring connectors 516 are provided for each pogo target 510. The spring connectors 516 in this example are soldered on their undersides to conducting pads on the PCB 500, with the upper spring portion providing the electrical connection to the pogo targets 510. This docking system provides both a mechanical and electrical connection for the PCB 500. In the example shown, there are six pogo pins 512 on the dock 300 which correspond to six pogo targets 510 on the device 220, each being arranged in two groups of three on opposite sides of the dock 300 and device 220 respectively. One group of three pogo pins 512 and pogo targets 516 in an example provide respectively for power, clock and ID (identification) connections. The ID connection may connect to an integrated circuit or chip on the (flexible) PCB 310 of the dock 300 discussed below that provides each dock 300 with its own unique ID for addressing purposes. The other group of three pogo pins 512 and pogo targets 516 in an example provide respectively for ground and for bus 1 and bus 2 which form a differential bus, as used in for example an RS 485 bus.

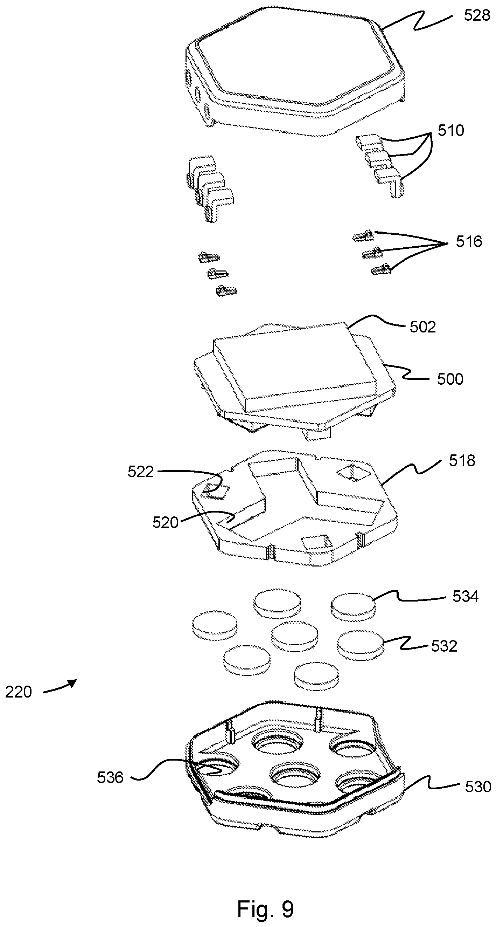

Referring particularly now to the exploded view of the device 220 in Figure 9, in an example, a black-out layer 518 is attached to the bottom surface of the PCB 500. The black-out layer 518 has a central cut-out 520 for the second part 504 of the Faraday cage and three peripheral cut-outs 522 for the light source units 222. The black-out layer 518 prevents light travelling directly from a light source unit 222 to a detector unit 224 within the device 220. In an example, the black-out layer 518 is made from silicone.

The PCB 500 and black-out layer 518 are enclosed in a two part housing 528, 530 of the device 220. The housing parts 528, 530 may be made of ABS for example. The two housing parts 528, 530 may for example be a push fit or may be clipped together. The top housing part 528 contains the pogo target mechanisms, including the pogo targets 510 and the spring connectors 516.

In an example, the bottom housing part 530 contains band pass interference filters 532, one for each light detector unit 224 and positioned underneath the respective light detector unit 224, which permit passage of light in the range emitted by the light source units 222. In a specific example, the band pass interference filters 532 for the light detector units 224 pass near-infra red light in the range 700 to 900 nanometres. In an example, the band pass interference filters 532 for the light detector units 224 are made from glass. A transparent cover 534 of for example a thin layer of ordinary glass is positioned under each light source unit 222. The bottom housing part 530 has apertures 536 for each light source unit 222 and each light detector unit 224 to allow light to exit and enter the housing. The band pass interference filters 532 and the covers 534 are a similar size and shape as the apertures 536 in the bottom housing part 530 which they cover and/or fill.

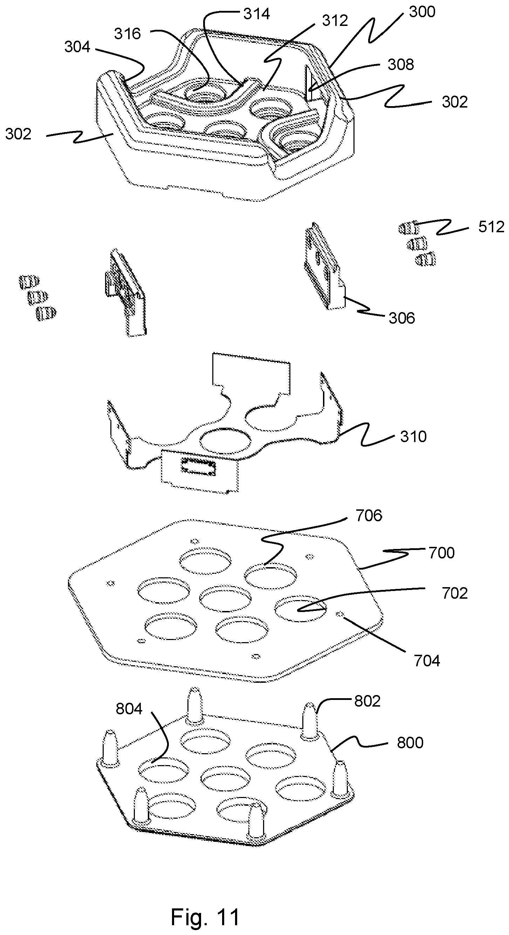

Referring now to Figure 11, this shows an exploded view of the example of the dock 300 discussed briefly above. The dock 300 removably receives a device 220 and acts as a mechanical support and retainer for the device 220. The dock 300 and the device 220 may be a friction fit or a snap fit, with ridges and recesses, etc. The dock 300 in this example also provides power and data connections for the device 220.

The dock 300 is generally in the form of an open container or receptacle with upstanding walls 302 and into which a device 220 can be passed. Two opposed sides 304 of the dock 300 are open, which allows a user to grip the device 220 to remove the device 220 from the dock 300 when required.

The pogo pins 512 of the dock 300, which provide the data and power connections with the device 220 via the pogo targets 5 10 of the device 220, in this example are carried by two pogo pin plates 306, each carrying three pogo pins 512. The pogo pin plates 306 are partially received in and retained by internally facing recesses 308 which are provided in opposed walls 302 of the dock 300. The pogo pins 512 face inwardly so as to engage the pogo targets 510 of the device 220 when the device 220 is inserted into the dock 300. In an example, a (flexible) PCB 310 is attached to the underside of the dock 300. The PCB 310 provides an electrical connection between the main serial bus and the six pogo pins 512 that connect to the device 220. It also provides for individually identifying each dock 300, via a suitable integrated circuit or chip mounted on the PCB 310 which has a unique ID stored in its on-board memory, as mentioned above. The main serial bus may connect to the front and rear tabs of the PCB 310 shown in Figure 11.

In an example, the internal face of the base wall 312 of the dock 300 has upstanding ridges 314 which help to prevent light travelling directly between light source units 222 and light detector units 224 of the device 220 received in the dock 300. In the example shown, there are three ridges 314, one round each region where a light source unit 222 is located when the device 220 is received in the dock 300. The base wall 312 also has seven through holes 316, one for each of the light source units 222 and light detector units 224 of the device 220, and which allow the light guide arrangement 400 to be clicked into place.

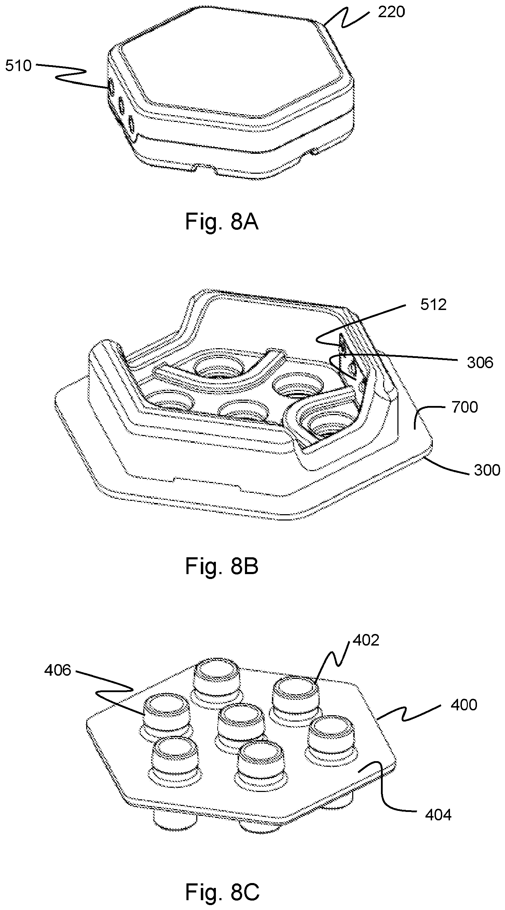

Referring particularly to Figure 8C, the light guide arrangement 400 comprises seven short lengths of light guides 402, in the form of optical fibres 402, one for each light source unit 222 and light detector unit 224 of the device 220, supported in and passing through a generally planar sheet 404. The planar sheet 404 may be made of for example compression moulded silicone. These light guides 402 channel the light from the light source units 222 to the tissue under investigation and back from the tissue to the light detector units 224.

Similarly to the example described above with reference to Figure 4, each light guide 402 in this example has an inner core and at least one coating or cladding layer. In a specific example, the light guide 402 has an inner core of for example (poly(methyl methacrylate) or PMMA). The inner core has a cladding of lower refractive index, made from polytetrafluoroethylene (PTFE). This helps to contain the light within the inner core. The outer coating is an opaque medium to prevent light from outside the light guide 402 passing into the light guide. The outer coating also prevents light transmission from within the light guide 402 to the outside of the light guide 402. The outer coating may be formed of for example transparent silicone mixed with a black, or substantially dark, pigment to provide an opaque surface. The black pigment may be for example charcoal powder. In another example, the outer coating may be formed of a material such as PC/ABS, which is a mixture of polycarbonate and acrylonitrile butadiene styrene. The outer coating may alternatively be formed of a material such as polypropylene or a fluorinated polymer.

Figure 11 also shows a portion of a retaining member or sheet 700, which may be a flexible sheet, such as a fabric. The retaining sheet 700 has a number of holes 702 corresponding to the light source units 222 and the light detector units 224. Figure 11 also shows a backing plate 800 for the dock 300 which will be discussed further below.

In an example, the device 220 is push-fitted into the dock 300. The connection between the pogo pins 512 on the dock 300 and the pogo targets 510 and the spring connectors 516 on the device 220 may be used to provide a secure connection for the device 220 in the dock 300. The light guide arrangement 400 is push-fitted into the main body of the dock 300 from the underside of the retaining sheet 700. The uppermost portions 406 of some or all of the light guides 402 may flare outwardly or be frustoconical or the like so as to engage with correspondingly shaped portions around the through holes 316 of the base wall 312 of the dock 300, thereby to retain the light guide arrangement 400 in close engagement with the dock 300.

Figure 12 shows an example of a portion of a measuring arrangement 900 for fitting to an animal body for measuring changes in concentration of a chromophore in the animal body. This example is in the form of a cap or headgear to be worn on the subject's head. Other configurations and arrangements are possible. For example, the measuring arrangement 900 may be in the form of a sleeve for fitting over a limb or a more simple sheet for placing over or wrapping round some other body part.

The measuring arrangement 900 has a sheet-like portion 700. (A portion of this corresponds to the retaining member or sheet 700 discussed with reference to Figure 11 above.) The sheet-like portion 700 may conveniently be a flexible, stretchable fabric. An example is neoprene.

A number of docks 300 for receiving devices 220 are arranged over the sheet-like member 700. The docks 300 are located at positions that correspond to the region(s) to be studied. In this example, the docks 300 are arranged so as to cover the entire cortex of the (human) subject. Each dock 300 is secured to the sheet-like member 700 in this example by first holding the backing plate 800 against the interior of the sheet-like member 700. As shown most clearly in Figure 11, the backing plates 800 have a number of upstanding projections or pins 802 which pass through the sheet-like member 700. Corresponding holes 704 may be provided in the sheet-like member 700 to receive the pins 802. In addition, holes 706 corresponding to the light guides 402 may also be provided in the sheet-like member 700. The docks 300 are then pushed up against the respective backing plates 800 from the exterior of the sheet-like member 700. The pins 802 of the backing plates 800 are received in and lock against recesses provided in the docks 300 in for example a snap-fit connection. The light guide arrangement 400 for each device 220 is then push-fitted into the main body of the dock 300 from the underside of the sheet-like member 700, the light guides 402 themselves passing through corresponding holes 804 in the backing plates 800.

In the example shown, a multi-conductor cable 910, acting as a bus, connects each dock 300 to the next dock 300 in turn. This allows all devices 220 received in use in the docks 300 to be controlled from a single electrical bus or cable 910. This means that the amount of electrical wiring needed in the imaging array is greatly reduced, again improving the signal-to-noise ratio. Nevertheless, other arrangements for data and power connections to and between the devices 220 are possible, as discussed above. The cable 910 may for example be located on top of the material of the sheet-like member 700 or sandwiched between two layers of the sheet-like member 700.

It may be noted that not all of the docks 300 have to be filled with a device 220. Moreover, the configuration of docks 300 that are filled with devices 220 may be varied, optionally on the fly during an investigation, to vary the image and the nature of the image that can be obtained.

This arrangement therefore provides for a measuring arrangement 900 that is flexible and (reasonably) comfortable for the subject of any study. A large number of devices 220 can be used, which improves the depth and quality generally of the measurements and images that can be obtained. The arrangement of the devices 220 can be easily and quickly changed. The light guide arrangement 400 for each device 220 can easily be removed, for example for cleaning either during use on a particular subject or prior to use on a different subject, and/or replaced.

In an example, the electrical serial bus multi-conductor cable 910 is terminated in a "stem" module which is positioned at the back of the measuring arrangement 900. This stem module may contain a circuit that converts the bus for the devices 220 (which may be for example a RS 485 bus) into a USB bus, which enables simple connection of the imaging array provided by the measuring arrangement 900 to a suitable laptop PC, which provides power, control and data collection for the entire imaging array.

In another example, the connecting module to the measuring arrangement 900 is known as a "hub" and provides more functionality than a "stem" module. (A similar base unit or hub 160 is discussed above in relation to the example shown in overview in Figure 1.) Such a hub may contain batteries to provide power to the measuring arrangement 900. The hub again provides bus conversion, in one example between the (RS 485) bus 900 and a suitable wireless link (180 in Figure 1) which in turn connects to a laptop PC or the like (170 in Figure 1). In an example, the hub may also contain an SD card for local storage of imaging data. The hub may have a user interface, consisting of for example buttons and LEDs, to enable user control and monitoring of basic functions. In an example the hub may also have a USB port to allow tethered operation, and a power socket to allow continuous operation without reliance on battery power.

There may also be provided a light guide for use with apparatus for measuring changes in concentration of a chromophore in a biological medium using at least one light source and at least one light detector, the light guide comprising:
an inner core;
an inner coating surrounding the inner core; and
an outer coating surrounding the inner coating; wherein:
   the inner core is a polymer that is transparent to light emitted by the light source of the apparatus,
   the inner coating is reflective to light transmitted by light emitted by the light source of the apparatus, and
   the outer coating is opaque to light emerging from the inner core and inner coating of the light guide. In an example, the inner core is optically clear silicone.

It is to be understood that any feature described in relation to any one embodiment may be used alone, or in combination with other features described, and may also be used in combination with one or more features of any other of the embodiments, or any combination of any other of the embodiments. Furthermore, modifications not described above may also be employed without departing from the scope of the invention, which is defined in the accompanying claims.

## Claims

1. A measuring apparatus (900) for fitting to an animal body for measuring changes in concentration of a chromophore in the animal body, the measuring apparatus (900) comprising:
a carrier (700) for fitting over the animal body;
a plurality of devices (220), each device (220) having a light source (223) for emitting light towards an animal body to which the carrier is fitted in use, a light detector (225) for detecting light returning from a said animal body, and a device controller (126) for receiving signals from and sending signals to a main controller (160);
wherein said carrier comprises
a plurality of docks (300) each for removably receiving a respective device (220) to mount the devices (220) to the carrier;
**characterised by**
an electrical connection arrangement provided at least in part by a shared bus (150, 910), atleast some of the docks (300)
comprising an electrical connector for making respective connections between the shared bus (910) and the device controller (126) of a device (220) received in the dock (300) to allow signals to be passed between the device controllers (126) of the plural devices (220) and a said main controller (160) via the bus.

2. A measuring apparatus according to claim 1, the shared bus (150, 910)
having plural connector ports to which the device controllers (126) of at least some of the devices (220) are respectively connected.

3. A measuring apparatus according to claim 2, wherein the shared bus (150) is a shared serial communication bus.

4. A measuring apparatus according to claim 2 or claim 3, wherein at least one of the devices (220) is connected to the shared bus (150) by flexible conductors (310).

5. A measuring apparatus according to any of claims 1 to 4, wherein the electrical connection arrangement is provided at least in part by a wiring scheme in which the plurality of devices (220) are connected in sequence.

6. A measuring apparatus according to any of claims 1 to 5, wherein the electrical connection arrangement is provided at least in part by a wireless connection between at least some of the devices (220).

7. A measuring apparatus according to any of claims 1 to 6 , wherein the electrical connector of said at least some of the docks (300) comprises a pogo pin (512) which connects to a corresponding pogo target (510) of a device (220) received in the dock (300).

8. A measuring apparatus according to any of claims 1 to 7, wherein the light source (223) of at least one device (220) is arranged to emit near-infrared light.

9. A measuring apparatus according to any of claims 1 to 8, wherein each of the plurality of devices (220) comprises at least two light sources (223) wherein a first light source (223) is arranged to emit light of a first wavelength and a second light source (223) is arranged to emit light of a second wavelength which is different from the first wavelength.

10. A measuring apparatus according to claim 9, wherein the distance between the at least two light sources (223) is no more than 15% of the distance between each of the at least two light sources (223) and the light detector (225).

11. A measuring apparatus according to any of claims 1 to 10, each of the plurality of devices (220) comprising:
a current-to-digital converter for the light detector (225), the current-to-digital converter having a transimpedance amplifier and an analogue-to-digital converter, wherein:
the transimpedance amplifier is arranged to produce a voltage output; and,
the analogue-to-digital converter is arranged to read the voltage output by the amplifier.

12. A measuring apparatus according to any of claims 1 to 11, each of the plurality of devices (220) comprising a substrate in which the light source (223), the light detector (225) and device controller (126) are arranged, the substrate being transparent to light emitted by the light source (223).

13. A measuring apparatus according to any of claims 1 to 12, each of the plurality of devices (220) comprising a plurality of light guides (140, 402), wherein:
a first light guide (140, 402) is arranged to guide light from the light source (223) to an animal body; and,
a second light guide (140, 402) is arranged to guide light from said animal body to the light detector (225).

14. A measuring apparatus according to claim 13, wherein at least one of: the first light guide (140, 402) is generally cylindrical and arranged around the light source (223); and the second light guide (140, 402) is generally cylindrical and arranged around the light detector (225).

15. A measuring apparatus according to any of claims 1 to 14, comprising:
a main controller (160) for sending signals to and receiving signals from the devices (220); and,
the shared bus being arranged to relay signals between the main controller (160) and the devices (220), the signals including at least one of light source intensity instructions, clock signals and data signals;
the main controller (160) being arranged to generate a system clock and initiate communication with the devices (220).

16. A measuring apparatus according to any of claims 1 to 15, wherein the devices (220) and the docks (300) have a hexagonal shape.

## Patentansprüche

1. Messvorrichtung (900) zum Aufsetzen auf einen Tierkörper zur Messung von Veränderungen einer Konzentration eines Chromophors in dem Tierkörper, wobei die Messvorrichtung (900) umfasst:
einen Träger (700) zum Aufsetzen über den Tierkörper;
eine Vielzahl von Vorrichtungen (220), wobei jede Vorrichtung (220) eine Lichtquelle (223) zum Abstrahlen von Licht zu einem Tierkörper hin, auf den der Träger aufgesetzt ist, im Gebrauch, einen Lichtdetektor (225) zum Erfassen von Licht, das von dem Tierkörper zurückkehrt, und eine Vorrichtungssteuerung (126) zum Empfangen von Signalen von und Senden von Signalen an eine Hauptsteuerung (160) aufweist;
wobei der Träger mehrere Docks (300) umfasst, die jeweils zum entfernbaren Aufnehmen einer jeweiligen Vorrichtung (220) sind, um die Vorrichtung (220) an dem Träger zu montieren;
**gekennzeichnet durch**
eine elektrische Verbindungsanordnung, die zumindest zum Teil von einem gemeinsam genutzten Bus (150, 910) bereitgestellt wird, wobei mindestens einige der Docks (300) einen elektrischen Verbinder zum Herstellen von jeweiligen Verbindungen zwischen dem gemeinsam genutzten Bus (910) und der Vorrichtungssteuerung (126) einer Vorrichtung (220), die in dem Dock (300) aufgenommen wird, umfassen, um zuzulassen, dass Signale zwischen den Vorrichtungssteuerungen (126) der mehreren Vorrichtungen (220) und der Hauptsteuerung (160) mittels dem Bus passieren gelassen werden.

2. Messvorrichtung nach Anspruch 1, wobei der gemeinsam genutzte Bus (150, 910) mehrere Verbinderanschlüsse aufweist, mit denen die Vorrichtungssteuerungen (126) von mindestens einigen der Vorrichtungen (220) jeweils verbunden werden.

3. Messvorrichtung nach Anspruch 2, wobei der gemeinsam genutzte Bus (150) ein gemeinsam genutzter Bus zur seriellen Kommunikation ist.

4. Messvorrichtung nach Anspruch 2 oder 3, wobei mindestens eine der Vorrichtungen (220) mit dem gemeinsam genutzten Bus (150) durch flexible Leiter (310) verbunden ist.

5. Messvorrichtung nach einem der Ansprüche 1 bis 4, wobei die elektrische Verbindungsanordnung zumindest zum Teil von einem Schaltplan bereitgestellt wird, in dem die Vielzahl von Vorrichtungen (220) hintereinander geschaltet sind.

6. Messvorrichtung nach einem der Ansprüche 1 bis 5, wobei die elektrische Verbindungsanordnung zumindest zum Teil von einer drahtlosen Verbindung zwischen mindestens einigen der Vorrichtungen (220) bereitgestellt wird.

7. Messvorrichtung nach einem der Ansprüche 1 bis 6, wobei der elektrische Verbinder der mindestens einigen der Docks (300) einen Federkontaktstift (512) umfasst, der sich mit einem entsprechenden Federkontaktziel (510) einer Vorrichtung (220), die in dem Dock (300) aufgenommen wird, verbindet.

8. Messvorrichtung nach einem der Ansprüche 1 bis 7, wobei die Lichtquelle (223) von mindestens einer Vorrichtung (220) dazu eingerichtet ist, Nah-Infrarotlicht abzustrahlen.

9. Messvorrichtung nach einem der Ansprüche 1 bis 8, wobei jede von der Vielzahl von Vorrichtungen (220) mindestens zwei Lichtquellen (223) umfasst, wobei eine erste Lichtquelle (223) dazu eingerichtet ist, Licht mit einer ersten Wellenlänge abzustrahlen, und eine zweite Lichtquelle (223) dazu eingerichtet ist, Licht mit einer zweiten Wellenlänge, die sich von der ersten Wellenlänge unterscheidet, abzustrahlen.

10. Messvorrichtung nach Anspruch 9, wobei der Abstand zwischen den mindestens zwei Lichtquellen (223) nicht mehr als 15 % des Abstands zwischen jeder von den mindestens zwei Lichtquellen (223) und dem Lichtdetektor (225) beträgt.

11. Messvorrichtung nach einem der Ansprüche 1 bis 10, wobei jede von der Vielzahl von Vorrichtungen (220) umfasst:
einen Strom-Digital-Wandler für den Lichtdetektor (225), wobei der Strom-Digital-Wandler einen Transimpedanzverstärker und einen Analog-Digital-Wandler aufweist, wobei:
der Transimpedanzverstärker dazu eingerichtet ist, einen Spannungsausgang zu produzieren; und
der Analog-Digital-Wandler dazu eingerichtet ist, den Spannungsausgang durch den Verstärker zu lesen.

12. Messvorrichtung nach einem der Ansprüche 1 bis 11, wobei jede von der Vielzahl von Vorrichtungen (220) ein Substrat umfasst, in dem die Lichtquelle (223), der Lichtdetektor (225) und die Vorrichtungssteuerung (126) eingerichtet sind, wobei das Substrat für Licht, das von der Lichtquelle (223) abgestrahlt wird, durchlässig ist.

13. Messvorrichtung nach einem der Ansprüche 1 bis 12, wobei jede von der Vielzahl von Vorrichtungen (220) eine Vielzahl von Lichtleitern (140, 402) umfasst, wobei:
ein erster Lichtleiter (140, 402) dazu eingerichtet ist, Licht von der Lichtquelle (223) zu einem Tierkörper zu leiten; und
ein zweiter Lichtleiter (140, 402) dazu eingerichtet ist, Licht von dem Tierkörper zu dem Lichtdetektor (225) zu leiten.

14. Messvorrichtung nach Anspruch 13, wobei mindestens eines von: der erste Lichtleiter (140, 402) ist allgemein zylindrisch und um die Lichtquelle (223) herum eingerichtet ist; und der zweite Lichtleiter (140, 402) allgemein zylindrisch ist und um den Lichtdetektor (225) herum eingerichtet ist.

15. Messvorrichtung nach einem der Ansprüche 1 bis 14, umfassend:
eine Hauptsteuerung (160) zum Senden von Signalen an und Empfangen von Signalen von den Vorrichtungen (220); und
wobei der gemeinsam genutzte Bus dazu eingerichtet ist, Signale zwischen der Hauptsteuerung (160) und den Vorrichtungen (220) weiterzuleiten, wobei die Signale mindestens eines von Lichtquellenintensitätsanweisungen, Taktsignalen und Datensignalen beinhalten;
wobei die Hauptsteuerung (160) dazu eingerichtet ist, einen Systemtakt zu erzeugen und eine Kommunikation mit den Vorrichtungen (220) zu initiieren.

16. Messvorrichtung nach einem der Ansprüche 1 bis 15, wobei die Vorrichtungen (220) und die Docks (300) eine hexagonale Form aufweisen.

## Revendications

1. Appareil de mesure (900) destiné à des fins de positionnement sur le corps d'un animal afin de mesurer des changements de concentration d'un chromophore dans le corps de l'animal, l'appareil de mesure (900) comportant :
un support (700) destiné à des fins de positionnement sur le corps de l'animal ;
une pluralité de dispositifs (220), chaque dispositif (220) ayant une source de lumière (223) servant à émettre de la lumière en direction du corps d'un animal sur lequel le support est positionné lors de l'utilisation, un détecteur de lumière (225) servant à détecter la lumière revenant en provenance dudit corps d'un animal, et un module de commande de dispositif (126) servant à recevoir des signaux en provenance d'un module de commande principal (160) et à envoyer des signaux à ce dernier ;
dans lequel ledit support comporte une pluralité de socles d'accueil (300) servant chacun à recevoir, de manière amovible, un dispositif respectif (200) à des fins de monture des dispositifs (220) sur le support ;
**caractérisé par** un agencement de connexion électrique mis en œuvre au moins en partie par un bus partagé (150, 910), au moins certains des socles d'accueil (300) comportant un connecteur électrique servant à la réalisation de connexions respectives entre le bus partagé (910) et le module de commande de dispositif (126) d'un dispositif (220) reçu dans le socle d'accueil (300) pour permettre à des signaux de passer entre les modules de commande de dispositif (126) de la pluralité de dispositifs (220) et un dit module de commande principal (160) par le biais du bus.

2. Appareil de mesure selon la revendication 1, le bus partagé (150, 910) ayant une pluralité de ports de connecteur auxquels les modules de commande de dispositif (126) d'au moins certains des dispositifs (220) sont connectés respectivement.

3. Appareil de mesure selon la revendication 2, dans lequel le bus partagé (150) est un bus de communication de série partagé.

4. Appareil de mesure selon la revendication 2 ou la revendication 3, dans lequel au moins l'un des dispositifs (220) est connecté au bus partagé (150) par des conducteurs flexibles (310) .

5. Appareil de mesure selon l'une quelconque des revendications 1 à 4, dans lequel l'agencement de connexion électrique est mis en œuvre au moins en partie par un schéma électrique dans lequel les dispositifs de la pluralité de dispositifs (220) sont connectés de manière séquentielle.

6. Appareil de mesure selon l'une quelconque des revendications 1 à 5, dans lequel l'agencement de connexion électrique est mis en œuvre au moins en partie par une connexion sans fil entre au moins certains des dispositifs (220).

7. Appareil de mesure selon l'une quelconque des revendications 1 à 6, dans lequel le connecteur électrique desdits au moins certains des socles d'accueil (300) comporte une broche pogo (512) qui se connecte à une cible pogo correspondante (510) d'un dispositif (220) reçu dans le socle d'accueil (300).

8. Appareil de mesure selon l'une quelconque des revendications 1 à 7, dans lequel la source de lumière (223) d'au moins un dispositif (220) est agencée pour émettre de la lumière infrarouge proche.

9. Appareil de mesure selon l'une quelconque des revendications 1 à 8, dans lequel chacun de la pluralité de dispositifs (220) comporte au moins deux sources de lumière (223), dans lequel une première source de lumière (223) est agencée pour émettre de la lumière d'une première longueur d'onde et une deuxième source de lumière (223) est agencée pour émettre de la lumière d'une deuxième longueur d'onde qui est différente de la première longueur d'onde.

10. Appareil de mesure selon la revendication 9, dans lequel la distance entre lesdites au moins deux sources de lumière (223) ne mesure pas plus de 15 % de la distance entre chacune desdites au moins deux sources de lumière (223) et le détecteur de lumière (225).

11. Appareil de mesure selon l'une quelconque des revendications 1 à 10, chacun de la pluralité de dispositifs (220) comportant :
un convertisseur de courant en numérique pour le détecteur de lumière (225), le convertisseur de courant en numérique ayant un amplificateur à transimpédance et un convertisseur analogique numérique, dans lequel :
l'amplificateur à transimpédance est agencé pour produire une tension de sortie ; et,
le convertisseur analogique numérique est agencé pour lire la tension émise en sortie par l'amplificateur.

12. Appareil de mesure selon l'une quelconque des revendications 1 à 11, chacun de la pluralité de dispositifs (220) comportant un substrat dans lequel la source de lumière (223), le détecteur de lumière (225) et le module de commande de dispositif (126) sont agencés, le substrat étant transparent par rapport à la lumière émise par la source de lumière (223).

13. Appareil de mesure selon l'une quelconque des revendications 1 à 12, chacun de la pluralité de dispositifs (220) comportant une pluralité de dispositifs de guidage de lumière (140, 402), dans lequel :
un premier dispositif de guidage de lumière (140, 402) est agencé pour guider de la lumière depuis la source de lumière (223) en direction du corps d'un animal ; et,
un deuxième dispositif de guidage de lumière (140, 402) est agencé pour guider de la lumière depuis ledit corps de l'animal en direction du détecteur de lumière (225).

14. Appareil de mesure selon la revendication 13, dans lequel au moins l'un parmi : le premier dispositif de guidage de lumière (140, 402) est généralement cylindrique et agencé autour de la source de lumière (223) ; et le deuxième dispositif de guidage de lumière (140, 402) est généralement cylindrique et agencé autour du détecteur de lumière (225).

15. Appareil de mesure selon l'une quelconque des revendications 1 à 14, comportant :
un module de commande principal (160) servant à envoyer des signaux aux dispositifs (220) et à recevoir des signaux en provenance de ceux-ci ; et
le bus partagé étant agencé pour relayer des signaux entre le module de commande principal (160) et les dispositifs (220), les signaux comprenant au moins l'un parmi des instructions d'intensité de la source de lumière, des signaux d'horloge et des signaux de données ;
le module de commande principal (160) étant agencé pour générer une horloge système et pour initialiser une communication avec les dispositifs (220).

16. Appareil de mesure selon l'une quelconque des revendications 1 à 15, dans lequel les dispositifs (220) et les socles d'accueil (300) ont une forme hexagonale.
